# EUROPEAN PATENT SPECIFICATION

(11) **EP 3 636 768 B1**
(45) Date of publication and mention of the grant of the patent: **16.03.2022**
(21) Application number: 19201844.8
(22) Date of filing: 08.10.2019
(51) Int. Cl.: C12Q 1/44

(54) **METHOD AND MEANS FOR DETERMINING THE BIODEGRADABILITY OF POLYMERIC MATERIALS**
VERFAHREN UND MITTEL ZUR BESTIMMUNG DER BIOLOGISCHEN ABBAUBARKEIT VON POLYMERMATERIALIEN
PROCÉDÉ ET MOYENS DE DÉTERMINATION DE LA BIODÉGRADABILITÉ DE MATÉRIAUX POLYMÈRES

(30) Priority: 08.10.2018 EP 18199071
(43) Date of publication of application: 15.04.2020
(73) Proprietor: B4PLastics BV, 3650 Dilsen-Stokkem (BE)
(72) Inventor: De Wildeman, Stefaan, 3630 Maasmechelen (BE); Elford, Matthew, 6229 GC Maastricht (NL)
(74) Representative: De Clercq & Partners

(56) References cited:
- WO-A2-2017/204615
- US-B1- 6 548 278

## Description

### FIELD OF THE INVENTION

The present invention relates to a method and means for determining and/or tracing the biodegradability of polymeric materials. In particular, the present invention provides a hydrogel composition for determining and/or tracing the biodegradability of polymeric materials. The present invention also provides a kit comprising said hydrogel composition. The present invention also provides a method for determining the degradability of polymeric materials by means of said hydrogel composition. The present invention also provides a computer-implemented method for determining the degradability of polymeric materials by means of said hydrogel composition.

### BACKGROUND

The intense use of modern polymeric materials, that is plastics, has created serious problems for waste management, with the main problems being caused by plastic packaging for consumer products. Indeed, their high durability is disproportionate to their short-term and single-use applications and is severely at odds with keeping a clean and sustainable world for future generations.

To provide for alternative waste management strategies, novel polymeric materials were developed, which ideally have performances that still fulfil usage needs and sometimes are comparable with that of conventional polymers but are also susceptible to (microbial or other) degradation when exposed to natural environments.

The degradability provides these materials with novel and additional properties which may also be beneficial after their use. For instance, after use, the plastic packaging of consumer products can be degraded from polymer backbones to oligo/monomeric fractions for novel plastics in an efficient and ecologically friendly manner, namely chemical recycling or plastic 'upcycling'.

Biodegradability of polymeric materials is known as complete chemical dissolution of these polymers into their monomeric compounds through the action of of enzymes derived from micro-organisms such as bacteria, fungi, or any other biological means. Ideally, these monomeric compounds are mineralized by living organisms and close the carbon cycle, producing newly generated biogas and biomass. Extracellular agents such as bio-surfactants and enzymes are known to enhance the overall biodegradation process.

However, there are many polymeric materials present on the consumer market and distinguishing the degradable from non-degradable can prove difficult. In fact, the consumer can only refer to the labelling on the plastic article, if any, although this labelling itself has been shown to be overly-complicated and therefore neglected or untrusted by the consumer. Moreover, even among the degradable plastic variants there exist different variations, which may require different processing steps or reaction products, such as enzymes. When improperly handled the polymeric materials may not fully degrade or degrade slower than expected, leading to waste build-up or other unwanted side-effects harming the planet in the long run.

The speed of degradation is typically dependent on parameters like temperature, humidity and microbial communities. Nowadays, plastic consumers are not at all in control to estimate degradation speed of their plastic waste in different conditions. Furthermore, missorting by consumers has proven to contribute to an average contamination level of 3,1% non-degradable plastics in envisioned biodegradable plastics fractions (in 2019), causing severe operational problems at conventional industrial waste composting sites. Recent studies and resulting statements of Plastics Associations indicate that there is a societal need to leverage the awareness and interest of the consumer to organize consumption behaviour and plastic collection in a more advanced way than the current practices. Laboratory testing can be performed to help distinguish which type of polymer is being handled, but these are often not user-friendly because the involved equipment is voluminous and expensive, while their handling is cumbersome and non-practical in the daily personal circumstances. Furthermore, analytical results of available equipment, such as FTIR for non-destructive measurements or NMR for destructive measurements, require highly educated insights in the field, relating analytical results with their interpretation, with the involved chemical composition of the materials, and consequently with their resulting degradation profile deduced from theoretical or empirical expertise. These tools are inaccessible to the average consumer.

US 6548278 relates to a process for enzymatic hydrolysis of cyclic oligomers of poly(ethylene terephthalate), which process comprises subjecting the cyclic oligomer to the action of one or more lipolytic and/or biopolyester hydrolytic enzyme(s).

WO 2017/2041615 relates to novel recombinant polynucleotides isolated from genes ancut1 and ancut2, which encode functional recombinant polypeptides that can degrade polyester plastics such as PET, of cutinase enzymes from Aspergillus nidulans.

There is a need for tools allowing for better distinction between degradable and non-degradable polymeric materials in a fast and cost-efficient way, preferably indicating the degradation speed and duration, in a way that can be performed in the field requiring little to no expertise or prior-knowledge.

### SUMMARY OF THE INVENTION

The devices and methods according to the present disclosure solve the aforementioned problems. Accordingly, provided herein are systems and methods for determining and/or tracing the biodegradability of polymeric materials.

In a first, the present invention relates to a method for determining and/or tracing the biodegradability of polymeric materials, the method comprising the steps of:
(i) providing a hydrogel composition comprising:
   - 0.05 to 10.0 wt.% of a cutinase;
   - a pH indicator suitable for visually indicating the cutinase's enzymatic activity, wherein the pH indicator is selected from bromothymol blue,bromocresol purple, litmus, anthocyanin, and/or methyl red;
   - a thickening agent comprising sodium carboxymethyl cellulose (CMC) present in an amount of at least 0.05 to at most 5.0 wt.%; and,
   - a buffer and/or pH adjustment agent comprising sodium bicarbonate (NaHCO3) and/or sodium hydroxide (NaOH) present in a concentration of at least 1.0 mM to at most 5.0 mM;
(ii) applying the hydrogel composition on a polymeric material; and,
(iii) determining and/or tracing the biodegradability of said polymeric material responsive to the visual indication of the biodegradability composition.

In particular, the method as disclosed herein is characterized in that the biodegradability composition is a hydrogel composition comprising cutinase; a pH indicator suitable for visually indicating the cutinase's enzymatic activity, preferably bromothymol blue and/or bromocresol purple; a thickening agent, preferably sodium carboxymethyl cellulose (CMC); a humectant; and, a buffer.

In particular, the method as disclosed herein is characterized in that the cutinase is chosen from *Thermobifida cellulosilytica* cutinase (Thc-cutl), *Thermobifida alba* cutinase (F71X06) and/or *Fusarium solani* cutinase, in particular *Fusarium solani* pisi cutinase (Cut1-Fusso).

In particular, the method as disclosed herein is characterized in that cutinase is present in a concentration in an amount of at least 0.01 to at most 10.0 wt.% of cutinase; preferably 0.05 to at 5.0 wt.%; preferably 0.05 to 4.0 wt.%, preferably 0.05 to 3.0 wt.%, more preferably 0.1 to 2.0 wt.%; more preferably 0.1 to 1.0 wt.%, even more preferably 0.2 to 0.9 wt.%, even more preferably 0.3 to 0.8 wt.%, even more preferably 0.3 to 0.7 wt.%; for example 0.4 wt.%, for example 0.5 wt.%, for example 0.6 wt.%.

In particular, the method as disclosed herein is characterized in that cutinase is present in a concentration of at least 0.1 to at most 1.0 wt.% of the total weight of the composition, preferably 0.1 to 0.9 wt.%, preferably 0.2 to 0.8 wt.%, more preferably 0.2 to 0.7 wt.%, more preferably 0.2 to 0.6 wt.%, even more preferably 0.3 to 0.6 wt.%, even more preferably 0.3 to 0.5 wt.%, for example 0.4 wt.%.

In particular, the method as disclosed herein is characterized in that the pH indicator is bromothymol blue, in particular wherein the composition has a pre-application (starting) pH of 7.5 to 8.5, preferably 7.8 to 8.5, preferably 7.9 to 8.4, preferably 8.0 to 8.4, more preferably 8.0 to 8.3, more preferably 8.0 to 8.2; for example 8.0; for example 8.1; for example 8.2.

In particular, the method as disclosed herein is characterized in that the pH indicator is bromocresol purple, in particular wherein the composition has a pre-application (starting) pH of 6.5 to 7.5; preferably 6.8 to 7.5; preferably 6.9 to 7.4; preferably 7.0 to 7.4; more preferably 7.0 to 7.2; for example 7.0; for example 7.1; for example 8.2.

In particular, the method as disclosed herein is characterized in that the thickening agent, preferably sodium carboxymethyl cellulose (CMC), is present in an amount of at least 0.01 to at most 10 wt.% of the total weight of the hydrogel composition; preferably 0.05 to 4.0 wt.%; preferably 0.05 to 3.0 wt.%; preferably 0.1 to 2.0 wt.%; preferably 0.1 to 1.0 wt.%; preferably 0.2 to 0.7 wt.%, more preferably 0.3 to 0.5 wt.%; for example 0.5 wt.%; for example 0.4 wt.%; for example 0.3 wt.%. In particular, the method as disclosed herein is characterized in that the buffer is sodium bicarbonate (NaHCO₃) in particular present in a concentration of at least 1.0 mM to at most 5.0 mM; preferably 2.0 mM to 4.5 mM; more preferably 3.0 mM to 4.0 mM; most preferably about 3.0 mM; and/or sodium hydroxide (NaOH) in particular present in a concentration of at least 1.0 mM to at most 5.0 mM; preferably 2.0 mM to 4.5 mM; more preferably 3.0 mM to 4.0 mM; most preferably about 4.0 mM.

In particular, the method as disclosed herein is characterized in that the biodegradability composition comprises a humectant, preferably propylene glycol. In particular wherein the humectant is present in at least 0.1 to at most 20.0 wt% of the total weight of the hydrogel composition; more preferably 1.0 to 10.0 wt%; more preferably 1.0 to 5.0 wt%; for example 3 wt%.

In particular, the method as disclosed herein is characterized in that the biodegradability composition comprises a stabilizer, preferably glycerol or sorbitol. In particular wherein the stabilizer is present in at least 5 to at most 50 wt% of the total weight of the composition; more preferably 10 to 40 wt%; more preferably 20 to 30 wt%; for example 25 wt%.

In a further aspect, the method as disclosed herein is characterized in that the method is a computer-implemented method for determining the biodegradability of a polymeric material, wherein step (iii) for determining and/or tracing the biodegradability further comprises the steps of:
(a) (after applying the biodegradability composition on a polymeric material,) receiving sensor data from an optical sensor unit configured for registering a degradation by the biodegradability composition of the polymeric material,
(b) computing, from the optical sensor unit data, a degradation speed of the polymeric material;
(c) computing, from the degradation rate, a biodegradability type of the polymeric material
(d) optionally, displaying the computed degradability type of the polymeric material on a display unit.

In particular, the method as disclosed herein is characterized in that the method comprises the preceding step of receiving user input on composition information and/or material information; wherein the composition information includes information on the enzyme type and/or concentration; and wherein the material information includes information on the polymer type and/or polymer colour.

In particular, the method as disclosed herein is characterized in that the sensor unit registers the degradation the polymeric material at predefined time intervals; preferably every 10-30 sec, more preferably 15-25 sec, most preferably approximately every 20 sec.

In particular, the method as disclosed herein is characterized in that the method comprises the step of alerting a user of the time interval for registering the degradation the polymeric material with the sensor unit; preferably every 1-10 sec before the registering, more preferably 3-8 sec, most preferably approximately 5 sec.

In a further aspect, disclosed herein is a kit for executing and/or facilitating the method of determining the biodegradability as disclosed herein, the kit comprising
- a hydrogel composition comprising
- 0.1 to 10.0 wt.% of a cutinase;
- a pH indicator suitable for visually indicating the cutinase's enzymatic activity, wherein the pH indicator is selected from bromothymol blue, bromocresol purple, litmus, anthocyanin, and/or methyl red;
- a thickening agent comprising sodium carboxymethyl cellulose (CMC) present in an amount of at least 0.05 to at most 5.0 wt.%; and,
- a buffer and/or pH adjustment agent comprising sodium bicarbonate (NaHCO3) and/or sodium hydroxide (NaOH) present in a concentration of at least 1.0 mM to at most 5.0 mM;
- an application means configured for applying the biodegradability composition on a polymeric material; and optionally,
- a system, preferably a personal computing hardware such as a smartphone, a tablet, or a phablet, comprising an optical sensor unit configured for registering photographic information from the color indicator, confining a degradation of the polymeric material by the composition, the system being configured for determining from data outputted by the sensor unit a biodegradability type of the polymeric material.

In particular, the kit as disclosed herein is characterized in that the application means are chosen from an adhesive plaster and/or a handheld pumping system or syringe.

In particular, the method as disclosed herein is characterized in that the application means is a 2 component (2K) system comprising
- a first component for holding the cutinase in a dry powdered form and configured for placement on the polymeric material; and,
- a second component for holding the buffer and pH indicator in a liquid form and configured for depositing on the first component.

In a further aspect, disclosed herein is a biodegradability composition for determining the biodegradability of polymeric materials comprising:
- 0.1 to 10.0 wt.% of a cutinase;
- a pH indicator suitable for visually indicating the cutinase's enzymatic activity, wherein the pH indicator is selected from bromothymol blue, bromocresol purple, litmus, anthocyanin, and/or methyl red;
- a thickening agent comprising sodium carboxymethyl cellulose (CMC) present in an amount of at least 0.05 to at most 5.0 wt.%; and,
- a buffer and/or pH adjustment agent comprising sodium bicarbonate (NaHCO3) and/or sodium hydroxide (NaOH) present in a concentration of at least 1.0 mM to at most 5.0 mM.

In particular the biodegradability composition as disclosed herein is characterized in that the biodegradability composition is a hydrogel composition comprising: a cutinase; a pH indicator suitable for visually indicating the cutinase's enzymatic activity, a thickening agent, and, a buffer.

In particular the biodegradability composition as disclosed herein is characterized in that the biodegradability composition is a hydrogel composition comprising: 0.01 to 10.0 wt.% of a cutinase; a pH indicator suitable for visually indicating the cutinase's enzymatic activity, preferably bromothymol blue and/or bromocresol purple; a thickening agent, preferably sodium carboxymethyl cellulose (CMC); and, a buffer and/or pH adjustment agent.

In particular the biodegradability composition as disclosed herein is characterized in that the biodegradability composition is a hydrogel composition comprising: 0.05 to 5.0 wt.% of a cutinase;a pH indicator suitable for visually indicating the cutinase's enzymatic activity, preferably bromothymol blue and/or bromocresol purple; a thickening agent, preferably sodium carboxymethyl cellulose (CMC); and, a buffer and/or pH adjustment agent.

In particular the biodegradability composition as disclosed herein is characterized in that the biodegradability composition is a hydrogel composition comprising: 0.1 to 1.0 wt.% of a cutinase;a pH indicator suitable for visually indicating the cutinase's enzymatic activity, preferably bromothymol blue and/or bromocresol purple; a thickening agent, preferably sodium carboxymethyl cellulose (CMC); and, a buffer and/or pH adjustment agent.

In particular, the composition as disclosed herein is characterized in that the composition comprises a humectant, preferably propylene glycol. In particular wherein the humectant is present in at least 0.1 to at most 20.0 wt% of the total weight of the hydrogel composition; more preferably 1.0 to 10.0 wt%; more preferably 1.0 to 5.0 wt%.

In particular, the composition as disclosed herein is characterized in that the composition comprises a stabilizer, preferably glycerol or sorbitol. In particular wherein the stabilizer is present in at least 5 to at most 50 wt% of the total weight of the composition; more preferably 10 to 40 wt%; more preferably 20 to 30 wt%.

In particular, the composition as disclosed herein is characterized in that the composition comprises a preservative, preferably phyenoxyethanol. In particular wherein the preservative is present in at least 0.05 to at most 2.0 wt% of the total weight of the hydrogel composition; preferably 0.1 to 1.5 wt%; preferably 0.1 to 1.0 wt%; more preferably 0.1 to 0.5 wt%.

### BRIEF DESCRIPTION OF THE DRAWINGS

The following description of the figures of specific embodiments of the invention is merely exemplary in nature and is not intended to limit the present teachings, their application or uses. Throughout the drawings, corresponding reference numerals indicate like or corresponding parts and features.
**Figure 1** illustrates testing performed on different types of polymeric materials for determining their biodegradability.
**Figure 2** illustrates exemplary application means that are regarded suitable for applying the biodegradability composition.

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In one or more aspects, the present invention relates to a means and method for determining the biodegradability of polymeric materials; in particular plastics in the form of thermoplastic or thermoset materials. It also relates to a computer-implemented method for determining the degradability of polymeric materials, a composition for use in said method, a kit for executing and/or facilitating said method and/or the use of the composition and/or kit in the method according to the present invention.The present invention may provide for highly effective, fast and cost-efficient determination of the biodegradability, and may also provide for an improved user-friendliness and accessibility.

Moreover, the means and method by the device or tool may allow for safely and accurately determining the biodegradability rate of polymeric materials without relying on third party information (e.g. catalogues, labels, certificates), which may be inaccurate, overwhelming or flawed. As a result, it may provide for higher responsibility of consumers, which may result in a more ecologically-friendly industrial and/or consumer consumption. The device or tool might therefore help to balance out a pushing socially responsible production apparatus, with a pulling socially responsible consumption counterforce.

Additionally, the means and method by the device or tool may be applied under various conditions, without relying on expensive and/or time-consuming analytical techniques (e.g. lab testing), and at any time, such as on location.

The term "polymeric materials" as used herein refers to organic materials consisting of multiple repeating subunits or monomers, typically created via a polymerization process. Polymeric materials are further optimized to become functional plastic materials by so-called compounding steps: often a customized set of additives and fillers is combined with specific processing conditions to result in a functional plastic article. Additives can be colorants, chemical stabilizers such as antioxidants, process stabilizing agents, nucleating agents or lubricants. Fillers can typically be inorganic salts or (micro)crystalline materials. Processing steps generally rely on extrusion equipment, injection moulding, or additive manufacturing methods. Resulting functionalities are optimized towards the specific application; i.e. towards wishes, norms and values in the market environment.

The term "(bio)degradation" as used herein refers to the process of (biologically) disintegrating materials by microorganisms, such as bacteria, fungi, or other biological means. Accordingly, a "(bio)degradable material" is a material that can be (biologically) disintegrated by microorganisms in a period of time, such as hours, days or weeks. The (bio)degradability of a material may be primarily determined by the presence of specific enzymes produced by the present microbial community that are capable of endo- or exo-cleaving of the polymeric backbones in the respective polymeric material. The biodegradability of a material can thereby be affected by a number of secondary factors that optimize the degradative capabilities by the present microbial community, such as temperature, pH, water and oxygen. Additionally, auxiliary factors may also influence the biodegradation, which can be intrinsic to the material itself such as crystallinity, surface roughness and bioavailability of polymeric chains or their released fragments, or be dependent on environmental conditions impacting the nature of the material such as light intensity and mechanical wear, such as (oceanic) waving or shaking. In some embodiments the degradability is related to the degradability of a polymeric material in an aqueous (marine) environment.

The combination of above-mentioned factors determines the overall rate of biodegradation. Methods for determining biodegradability and biodegradability rate under anaerobic and aerobic conditions are given in the following ASTM methods:
- ASTM D5210-07 "Standard Test Method for Determining the Anaerobic Biodegradation of Plastic Materials in the Presence of Municipal Sewage Sludge"
- ASTM D5338-15 "Standard Test Method for Determining Aerobic Biodegradation of Plastic Materials Under Controlled Composting Conditions, Incorporating Thermophilic Temperatures"
- ASTM D5511-12 "Anaerobic Biodegradation of Plastic Materials in a High Solids Environment Under High-Solids Anaerobic-Digestion Conditions"
- ASTM D5526-12 "Standard Test Method for Determining Anaerobic Biodegradation of Plastic Materials Under Accelerated Landfill Conditions"

For the purposes of the present invention and according to these test methods, a polymeric material is considered biodegradable when a defined fraction is removed by mineralization to CO₂ and cell mass growth within a defined timeframe under a defined set of (environmental) conditions. If it fails to reach the defined threshold values contained in the respective test method, it is not qualifying up to standard set forth by the method. Examples of biodegradable polymeric materials are polybutylene succinate (PBS), polybutylene co-adipate-terephthalate (PBAT), polyhydroxyalkanoates (PHAs) such as poly-3-hydroxybutyrate (PHB), polyhydroxyvalerate (PHV) and polyhydroxyhexanoate (PHH), polylactic acid (PLA), polycaprolactone (PCL), polybutylene sebacate, polyanhydrides, polyvinyl alcohol, cellulose esters, such as cellulose acetate and nitrocellulose and their derivatives, and/or polyethylene terephthalate. Examples of biodegradable polymeric materials can also be starch filled to enhance the degradability rate.

Several of the above thermoplastic material families have been granted biodegradation labels by independent certification agencies (e.g. Vincotte) based on the above mentioned ASTM methods.

The term "carboxylic ester hydrolase" as used herein (Enzyme Commission number = 3.1.1) refers to an enzyme that catalyzes the hydrolysis of a chemical bond. Accordingly the activity of carboxylic ester hydrolase refers to the catalysis of the hydrolysis of a chemical bond by said carboxylic ester hydrolase. As used herein the term "cutinases" refers to a specific set of enzymes belonging to the α/β-hydrolase superfamily (Enzyme Commission number = 3.1.1.74). Cutinase are capable of catalyzing the cleavage of cutin, which is a polymeric structural component of plant cuticles. There exist two major monomer families of cutin, the C16 and C18 families that contain one to three hydroxyl groups. Cutin is thereby embedded and overlaid by intracuticular and epicuticular waxes, complex mixtures of hydrophobic material containing very long-chain fatty acids and their derivatives. The combination of cutin, waxes and partially polysaccharides, forms the cuticle. Cuticles may be of lamellate, recticulate, or amorphous appearance. It is the extraordinary achievement of cutinases as hydrolytic enzymes to approach these complex hydrophobic material structures sufficiently with their catalytic center and mobilize water to cleave ester bonds initiating degradation of the cuticle layer. Cutinase is therefore the essential catalytic bridge between hydrophobic substrate and hydrophilic agent to create released and degraded material as a result. Furthermore, the cutinase is also able to adapt its catalytic conversion to more-or-less structured layers, translating in its ability to cope with semi-crystalline synthetic polymeric materials. The term "pH indicator" as used herein refers to a chemical compound added to a composition to allow visual determination of the pH (acidity or basicity). Accordingly, if carboxylic ester hydrolase's activity results in a change of the composition's pH value, the pH indicator will allow for visual determining of said carboxylic ester hydrolase's activity.

In a first aspect, the present invention relates to a method for determining the biodegradability of polymeric materials, the method comprising the steps of:
(i) providing a hydrogel composition comprising:
   - 0.05 to 10.0 wt.% of a cutinase;
   - a pH indicator suitable for visually indicating the cutinase's enzymatic activity, wherein the pH indicator is selected from bromothymol blue,bromocresol purple, litmus, anthocyanin, and/or methyl red;
   - a thickening agent comprising sodium carboxymethyl cellulose (CMC) present in an amount of at least 0.05 to at most 5.0 wt.%; and,
   - a buffer and/or pH adjustment agent comprising sodium bicarbonate (NaHCO3) and/or sodium hydroxide (NaOH) present in a concentration of at least 1.0 mM to at most 5.0 mM;
(ii) applying the hydrogel composition on a polymeric material; and,
(iii) determining and/or tracing the biodegradability of said polymeric material responsive to the visual indication of the biodegradability composition.

The determining as used herein preferably refers to a typically immediate or short-term response, preferably determining the biodegradability in a matter of minutes to even seconds. This is in sharp contrast to other techniques in the art wherein the biodegradability is determined over hours or even days. The determining may refer to establishing the biodegradability of an unknown polymeric material, to verifying the biodegradability of a known polymeric material (e.g. by verifying the composition of the polymeric material), and/or to tracing the biodegradability of a polymeric material (e.g. tracing how the biodegradability changes during polymer processing).

While the use of cutinase in a method and composition for determining the biodegradability of polymeric materials is particularly disclosed herein, it should be noted that also other types of enzymes could be used for the same purpose, in particular depending on the types of plastics. Carboxylic ester hydrolases such as cutinases can for instance be used for detecting biodegradable polyesters. PHB depolymerases can for instance be used for detecting biodegradable polyhydroxyalkanoates, using for instance methyl red or bromocresol purple as pH indicator. For starch-based plastics a composition comprising amylase and/or cellulase in combination with glucose detection means for determining biodegradability. The glucose detection means could for instance include glucose oxidase and peroxidase in combination with a color changing chromogen, a glucose dehydrogenase and an oxidized cofactor NAD+, and/or the detection of glucose through glucose strips known in the art. The enzyme may be provided in liquid or aqueous form, or alternatively a dry or powdered form which may be dissolved before or during the determining step. An example of a liquid form is a liquid enzyme concentrate. An example of a dry form is a freeze-dried or spray-dried enzyme powder.These systems typically work in the general conditions as disclosed herein. In particular, the method as disclosed herein is characterized in that the cutinase is chosen from *Thermobifida cellulosilytica* cutinase (Thc-cutl), *Thermobifida alba* cutinase (F71X06) and/or *Fusarium solani* cutinase, in particular *Fusarium solani* pisi cutinase (Cut1-Fusso). Preferably, the cutinase is *Fusarium solani* pisi cutinase (Cut1-Fusso).

The method as disclosed herein is characterized in that the cutinase is present in a concentration of at least 0.01 to at most 10.0 wt.% of the total weight of the composition, preferably 0.05 to most 10.0 wt.%, more preferably 0.1 to most 10.0 wt.%. In particular, the method as disclosed herein is characterized in that cutinase is present in a concentration in an amount of at least 0.05 to at most 5.0 wt.%; preferably 0.05 to 4.0 wt.%, preferably 0.05 to 3.0 wt.%, more preferably 0.1 to 2.0 wt.%; more preferably 0.1 to 1.0 wt.%, even more preferably 0.2 to 0.9 wt.%, even more preferably 0.3 to 0.8 wt.%, even more preferably 0.3 to 0.7 wt.%; for example 0.4 wt.%, for example 0.5 wt.%, for example 0.6 wt.%. More in particular, the method as disclosed herein is characterized in that cutinase is present in a concentration of at least 0.1 to at most 1.0 wt.% of the total weight of the composition, preferably 0.1 to 0.9 wt.%, preferably 0.2 to 0.8 wt.%, more preferably 0.2 to 0.7 wt.%, more preferably 0.2 to 0.6 wt.%, even more preferably 0.3 to 0.6 wt.%, even more preferably 0.3 to 0.5 wt.%, for example 0.4 wt.%.

The method as disclosed herein is characterized in that the cutinase is present in a concentration of at least 0.01 to at most 10.0 wt.% of the total weight of the composition, preferably 0.05 to most 10.0 wt.%, more preferably 0.1 to most 10.0 wt.%. In particular, the method as disclosed herein is characterized in that cutinase is present in a concentration in an amount of at least 0.05 to at most 5.0 wt.%; preferably 0.05 to 4.0 wt.%, preferably 0.05 to 3.0 wt.%, more preferably 0.1 to 2.0 wt.%; more preferably 0.1 to 1.5 wt.%, more preferably 0.1 to 1.0 wt.%, even more preferably 0.2 to 0.9 wt.%, even more preferably 0.3 to 0.8 wt.%, even more preferably 0.3 to 0.7 wt.%; for example 0.4 wt.%, for example 0.5 wt.%, for example 0.6 wt.%. More in particular, the method as disclosed herein is characterized in that cutinase is present in a concentration of at least 0.1 to at most 1.0 wt.% of the total weight of the composition, preferably 0.1 to 0.9 wt.%, preferably 0.2 to 0.8 wt.%, more preferably 0.2 to 0.7 wt.%, more preferably 0.2 to 0.6 wt.%, even more preferably 0.3 to 0.6 wt.%, even more preferably 0.3 to 0.5 wt.%, for example 0.4 wt.%.

The method as disclosed herein is characterized in that the cutinase is present in an amount of at least 0.01 g/L to at most 100 g/L of the total weight of the composition, preferably 0.05 g/L to most 100 g/L, more preferably 0.1 g/L to most 100 g/L. In particular, the method as disclosed herein is characterized in that cutinase is present in a concentration in an amount of at least 0.5 g/L to at most 50 g/L; preferably 0.5 g/L to 40 g/L, preferably 0.5 g/L to 30 g/L; more preferably 1 g/L to 20 g/L; more preferably 1 g/L to 15 g/L; more preferably 1 g/L to 10 g/L; even more preferably 2 g/L to 9 g/L, even more preferably 2 g/L to 8 g/L, even more preferably 3 g/L to 7 g/L; for example 4 g/L, for example 5 g/L for example 6 g/L.

In some embodiments the pH indicator is bromothymol blue, litmus, bromocresol purple, anthocyanin preferably is bromothymol blue and/or bromocresol purple. In particular, the method as disclosed herein is characterized in that the pH indicator is bromothymol blue, in particular wherein the composition has a starting pH of 7.5 to 8.5,, preferably 7.8 to 8.5, preferably 7.9 to 8.4, preferably preferably 8.0 to 8.4, preferably 8.1 to 8.4, more preferably 8.0 to 8.2; for example 8.0; for example 8.1; for example 8.2. Below 8.0 pH the colour may be less intensily blue, while above 8.2 the buffering capacity may be too high leading to reduced colour changing properties. In particular, the method as disclosed herein is characterized in that the pH indicator is bromocresol purple, in particular wherein the composition has a pre-application (starting) pH of 6.5 to 7.5; preferably 6.8 to 7.5; preferably 6.9 to 7.4; preferably 7.0 to 7.4; more preferably 7.0 to 7.2; for example 7.0; for example 7.1; for example 8.2. Below 7.0 pH the colour may be less intensily purple, while above 8.2 the buffering capacity may be too high leading to reduced colour changing properties. In particular, the method as disclosed herein is characterized in that the pH indicator is anthocyanin, in particular wherein the composition has a starting pH of 6.5 to 7.5, preferably 6.6 to 7.4, preferably 6.7 to 7.3, preferably 6.8 to 7.2, preferably 6.9 to 7.1; for example 7.0. Anthocyanin can produce a range of colours over a wide pH response range; the preferred pH range may allow for more reliable colour control and stability foreadout In particular, the method as disclosed herein is characterized in that the pH indicator is litmus, in particular wherein the composition has a starting pH of 8.0 to 8.5, preferably 8.1 to 8.4, more preferably about 8.2.

In some embodiments the biodegradability composition further comprises an aqueous medium (water). In some embodiments biodegradability composition further comprises a hydrogel. A hydrogel refers to a network of typically hydrophilic polymers that hold a large amount of aqueous medium (water) while maintaining the structure due to chemical or physical cross-linking of individual polymer chains. Hydrogels are typically formed from biopolymers and/or polyelectrolytes. Some exemplary hydrogel production methods are discussed further in the description. The skilled person, however, understands that various production methods can be used to produce a suitable hydrogel, and the present invention is not limited to one method in particular.

In some embodiments the biodegradability composition, preferably the hydrogel, further comprises a thickener or thickening agent. Thickening agent may be added to create the gelling characteristics of a hydrogel. In some embodiments the thickening agent is poly(acrylic acid), hydroxyethyl cellulose (HEC), and/or sodium Carboxymethyl cellulose (CMC). Other thickening agents, such as agarose, may be less suitable as thickening agent in the current hydrogel because of their elevated biodegradation tendency in contact with enzymes. Preferably, the thickening agent is present in an amount of at least 0.01 to at most 5.0 wt.% of the total weight of the hydrogel composition; preferably 0.05 to 4.0 wt.%; preferably 0.05 to 3.0 wt.%; preferably 0.05 to 2.5 wt.%; more preferably 0.1 to 2.0 wt.%; more preferably 0.1 to 1.5 wt.%; more preferably 0.1 to 1.0 wt.%; more preferably 0.1 to 0.9 wt.%, more preferably 0.2 to 0.8 wt.%, more preferably 0.2 to 0.7 wt.%, even more preferably 0.3 to 0.6 wt.%, even more preferably 0.3 to 0.5 wt.%; for example 0.5 wt.%; for example 0.4 wt.%; for example 0.3 wt.%. The preferred concentration of thickening agent may be dependent on the composition and the production method used. For instance, the initial concentation of the thickening agent may be higher, for example 0.5 wt%, and then be diluted when mixed with other components to reach a lower concentration, for example 0.3 wt%.

In some preferred embodiment the thickening agent is sodium Carboxymethyl cellulose (CMC). The inclusion of CMC may produce a particularly stable hydrogel that may allow other components to be incorporated without any of fewer adverse effects. It was found that standard thickening agents had various limitations with regards to the stability, longevity, viscosity, and/or processing difficulty of the biodegradability composition. For instance, the incorporation of, (predominantly acidic) cutinase may cause a pH decrease, depending on the thickening agent used, potentially causing a disruption in the cross-linking of the agent's chain network and decreasing the viscosity of the hydrogel. This may require the addition of a neutralisation agent to achieve suitable thickening. Moreover, hydrogels produced based on a standard thickening agent may exhibit thermal instability, requiring cooling when stored for a prolonged period of time (e.g. refrigerated storage for a week). CMC was unexpectedly found to solve the above mentioned problems. In particular, CMC may require less of no addition of neutralisation agents when adding cutinase to achieve a suitable pH value. Moreover, CMC may be show prolonged thermal stability making it suitable for use in a diagnostic kit as described herein.

In some embodiments the biodegradability composition, preferably the hydrogel, further comprises a humectant or wetting agent. In some embodiments the humectant is propylene glycol, hexylene glycol, butylene glycol, and/or a sugar alcohol, such as glycerol, sorbitol, xylitol, maltitol; preferably is propylene glycol. Additionally, the humectant may serve as a solvent or co-solvent in the composition. Preferably, the humectant is present in at least 0.1 to at most 20.0 wt% of the total weight of the composition, preferably the hydrogel; more preferably 1.0 to 10.0 wt%; more preferably 1.0 to 5.0 wt%; for example 2.0 wt%; for example 3.0 wt%; for example 4.0 wt%.

In some embodiments the biodegradability composition, preferably the hydrogel, further comprises a preservative, such as a germicide or disinfectant. In some embodiments the preservative is phyenoxyethanol. Preferably, the preservative is present in at least 0.05 to at most 2.0 wt% of the total weight of the composition, preferably the hydrogel; more preferably 0.1 to 1.5 wt%; more preferably 0.1 to 1.0 wt%; more preferably 0.1 to 0.7 wt%; more preferably 0.1 to 0.6 wt%; even more preferably 0.1 to 0.5 wt%; for example 0.2 wt%; for example 0.3 wt%; for example 0.4 wt%.

In some embodiments the biodegradability composition further comprises a buffer. The buffer is preferably used to maintain the pH of the biodegradability composition at a pH-value suitable for the pH indicatorr; it is a pH buffer. Preferably the buffer has a pH of at least 6.5 to at most 11.0; preferably 7.0 to 10.0; more preferably 7.5 to 9.5; most preferably 8.0 g/l to 9.0 g/l; for example 8.2; for example 8.5. In some embodiments the biodegradability composition further comprises a buffer pH adjustment agent. The pH adjustment agent is preferably used to adjust (increase or lower) the pH of the biodegradability composition to a pH-value suitable for the pH indicator. The pH adjustment agent may be part of the pH buffer system, or it may be an optional separate component.

In some embodiments the buffer and/or the pH adjustment agent may be contained in the hydrogel; the hydrogel may contain an immobilized pH buffer. In some embodiments the buffer is sodium bicarbonate (NaHCO₃); preferably present in a concentration of at least 1.0 mM to at most 5.0 mM; preferably 2.0 mM to 4.5 mM; more preferably 3.0 mM to 4.0 mM; most preferably about 3.0 mM; and/or sodium hydroxide (NaOH) in particular present in a concentration of at least 1.0 mM to at most 5.0 mM; preferably 2.0 mM to 4.5 mM; more preferably 3.0 mM to 4.0 mM; most preferably about 4.0 mM. In some embodiments the buffer is TRIS buffer, Kpi (phosphate) buffer, and/or acetate buffer.

In some embodiments the biodegradability composition, preferably the hydrogel, further comprises a stabilizer or stabilizing agent. Additionally, the stabilizer may serve as a solvent or co-solvent in the composition; in particular in embodiments wherein the enzyme is added in dry powder form. In some embodiments the stabilizer is glycerol, sorbitol, xylitol, maltitol; preferably is glycerol. Glycerol in particular was shown to be beneficial for achieving enzyme stabilization and preventing aggregation. In some preferred embodiments the biodegradability composition comprises glycerol or sorbitol in at least 5 to at most 50 wt% of the total weight of the composition; more preferably 10 to 40 wt%; more preferably 20 to 30 wt%; for example 25 wt%.

In some embodiments the composition further comprises stabilizers or processing additives, such as salts and/or organics, present in a concentration suitable for stabilizing the cutinase. The addition of additives may be a result of the enzyme processing steps, for instance when the enzyme is provided in a powder form.

In some embodiments the polymeric material (i.e. for determining the biodegradability) comprises cutin polymers. Cutin consists of omega hydroxy acids and their derivatives, which are interlinked via ester bonds, forming a polyester polymer of indeterminate size. In some embodiments the polymeric material is selected from the following list: polybutylene succinate (PBS), polybutylene adipate terephthalate (PBAT), polyhydroxyalkanoates (PHAs) such as poly-3-hydroxybutyrate (PHB), polyhydroxyvalerate (PHV) and polyhydroxyhexanoate (PHH), polylactic acid (PLA), polycaprolactone (PCL), polybutylene sebacate, polyanhydrides, polyvinyl alcohol, cellulose esters, such as cellulose acetate and nitrocellulose and their derivatives, and/or polyethylene terephthalate.

In a particularly preferred embodiment, the method comprises providing a biodegradability composition contained in a hydrogel. In some embodiments the hydrogel composition comprises: a cutinase, a pH indicator, a thickening agent, and a buffer and/or pH adjustment agent. Optionally, the hydrogel composition may comprise a a humectant, a preservative and/or a stabiliser.

In some embodiments the hydrogel composition comprises:
- at least 0.01 to at most 10.0 wt.% of a cutinase;
- at least 0.01 to at most 1.0 wt.% of a pH indicator;
- at least 0.05 to at most 5.0 wt.% of a thickening agent;
- at least 0.1 to at most 15.0 wt.% of a pH buffer and/or pH adjustment agent;
- optionally,- at least 0.1 to at most 15.0 wt.% of a humectant;
- optionally, at least 0.05 to at most 2.0 wt.% of a preservative; and,
- optionally, at least 10 to at most 40 wt.% of a stabiliser.

In a particularly preferred embodiment, the hydrogel composition comprises:
- at least 0.2 to at most 2.0 wt.% of a cutinase;
- at least 0.01 to at most 0.1 wt.% of a pH indicator;
- at least 0.01 to at most 2.0 wt.% of a thickening agent;
- at least 0.5 to at most 10.0 wt.% of a pH buffer and/or pH adjustment agent
- optionally, at least 1.0 to at most 10.0 wt.% of a humectant;
- optionally, at least 0.1 to at most 1.0 wt.% of a preservative; and,
- optionally, at least 15 to at most 35 wt.% of a stabiliser.ln a particularly preferred embodiment, the hydrogel composition comprises
- at least 0.3 to at most 1.0 wt.% of a cutinase;
- at least 0.01 to at most 0.05 wt.% of a pH indicator;
- at least 0.1 to at most 1.0 wt.% of a thickening agent;
- at least 1.0 to at most 10.0 wt.% of a pH buffer and/or pH adjustment agent;
- optionally, at least 1.0 to at most 5.0 wt.% of a humectant;
- optionally, at least 0.1 to at most 0.5 wt.% of a preservative; and,
- optionally, at least 20 to at most 30 wt.% of a stabiliser.

In some embodiments the hydrogel composition comprises:
- at least 0.01 to at most 10.0 wt.% of Fusarium solani pisi cutinase (Cut1-Fusso);
- at least 0.01 to at most 1.0 wt.% of bromothymol blue and/or bromocresol purple;
- at least 0.05 to at most 5.0 wt.% of carboxymethyl cellulose;
- at least 0.1 to at most 15.0 wt.% of NaOH and/or NAHCO₃;
- optionally,- at least 0.1 to at most 15.0 wt.% of propylene glycol;
- optionally, at least 0.1 to at most 2.0 wt.% of a phyenoxyethanol; and,,
- optionally, at least 10 to at most 40 wt.% of glycerol and/or sorbitol.

In a particularly preferred embodiment, the hydrogel composition comprises:
- at least 0.2 to at most 2.0 wt.% of Fusarium solani pisi cutinase (Cut1-Fusso);
- at least 0.01 to at most 0.1 wt.% bromothymol blue and/or bromocresol purple;
- at least 0.01 to at most 2.0 wt.% of carboxymethyl cellulose;
- at least 0.5 to at most 10.0 wt.% of NaOH and/or NAHCO₃
- optionally, at least 1.0 to at most 10.0 wt.% of propylene glycol;
- optionally, at least 0.1 to at most 1.0 wt.% of a phyenoxyethanol; and,
- optionally, at least 15 to at most 35 wt.% of glycerol and/or sorbitol.

In a particularly preferred embodiment, the hydrogel composition comprises
- at least 0.3 to at most 1.0 wt.% of Fusarium solani pisi cutinase (Cut1-Fusso);
- at least 0.01 to at most 0.05 wt.% of bromothymol blue and/or bromocresol purple;
- at least 0.1 to at most 1.0 wt.% of carboxymethyl cellulose;
- at least 1.0 to at most 10.0 wt.% of NaOH and/or NAHCO₃;
- optionally, at least 1.0 to at most 5.0 wt.% of propylene glycol;
- optionally, at least 0.1 to at most 0.5 wt.% of a phyenoxyethanol; and,
- optionally, at least 20 to at most 30 wt.% of glycerol and/or sorbitol.

In a further aspect, the method as disclosed herein is characterized in that the method is a computer-implemented method for determining the biodegradability of a polymeric material further comprising the steps:
(a) after applying the biodegradability composition on a polymeric material, receiving sensor data from an optical sensor unit configured for registering a degradation by the biodegradability composition of the polymeric material,
(b) computing, from the optical sensor unit data, a degradation speed of the polymeric material;
(c) computing, from the degradation rate, a biodegradability type of the polymeric material;
(d) optionally, displaying the computed degradability type of the polymeric material on a display unit.

Preferably, the above computer-implemented method replaces step (iii) of the method for determining and/or tracing the biodegradability of a polymeric material as described herein; and/or is performed after step (ii) of the method for determining and/or tracing the biodegradability of a polymeric material as described herein.

As used herein the "degradation speed" refers to the speed at which the cutinase catalyses the polymeric material (by cleaving ester bonds) upon contact. Alternatively degradation speed may be understood to refer to catalytic speed or enzymatic activity.

As used herein the "degradability type" refers to the type of polymeric material with regards to its degradability in a natural or aqueous environment.

In some embodiments the degradability type includes at least the following classes:
- (Bio)degradable polymeric material;
- Slow or doubtful (bio)degradable polymeric material;
- Non-(bio)degradable polymeric material.

The sensor data outputted by the sensor unit may be extracted, converted and analysed by a computing unit. The sensor data may include relative or absolute colourings of the composition, in particular colour changes of the pH indicator. The sensor unit may or may not require prior input of information from a user of the system. For instance, for determining biodegradability the user may be requested to enter information on the composition information, such as information on the enzyme type and/or enzyme concentration, and/or material information, such as such as information on the polymer type and/or polymer colour. Additionally or alternatively, the composition information and/or material information may be automatically determined by the sensor unit prior to performing the method. The sensor unit data may be configured for scanning an optical, machine-readable, representation of data corresponding with the composition information; for example, the user may be prompted to scan a barcode which includes information on the enzyme type and/or enzyme concentration. The sensor unit data may also be configured to determine the colour of the polymer from the surrounding area, i.e. the area not contacted with the degradability composition.

The optical sensor unit is configured for determining the colouring changes of the pH indicator. The optical sensor unit may be a camera, such as a CCD and CMOS camera.

The display sensor unit is configured for displaying the computed or determined degradability type of the polymeric material. The display unit may be a visual display, such as an LED or LCD screen. Alternatively, other means of notifying a user may be provided, like an audio signal playing, or a vibration of the device, and so on. The skilled person understands that for the present invention the notification of the computed or determined degradability type is relevant, and not the means of notifying said information.

In particular, the method as disclosed herein is characterized in that the method comprises the preceding step of receiving user input on composition information and/or material information; wherein the composition information includes information on the enzyme type and/or concentration; and wherein the material information includes information on the polymer type and/or polymer colour.

In particular, the method as disclosed herein is characterized in that the sensor unit registers the degradation the polymeric material at predefined time intervals; preferably every 10-30 sec, more preferably 15-25 sec, most preferably approximately every 20 sec.

In particular, the method as disclosed herein is characterized in that the method comprises the step of alerting a user of the time interval for registering the degradation the polymeric material with the sensor unit; preferably every 1-10 sec before the registering, more preferably 3-8 sec, most preferably approximately 5 sec.

In some embodiments the alerting is an audio cue provided by an audio unit and/or a visual cue provided by a display unit.

In some embodiments wherein the degradability type includes at least the following classes:
- (bio)degradable polymeric material;
- rather (bio)degradable polymeric material
- slow or doubtful (bio)degradable polymeric material;
- rather non-(bio)degradable polymeric material
- non-(bio)degradable polymeric material.

In some embodiments the degradability type is related to the degradability of a polymeric material in an aqueous (marine) environment.

In a further aspect, disclosed herein is a kit for executing and/or facilitating the method as disclosed herein, the kit comprising
a hydrogel composition comprising
   - 0.1 to 10.0 wt.% of a cutinase, preferably chosen from *Thermobifida cellulosilytica* cutinase (Thc-cutl), *Thermobifida alba* cutinase (F71X06) and/or *Fusarium solani* cutinase, in particular *Fusarium solani* pisi cutinase (Cut1-Fusso);
   - a pH indicator suitable for visually indicating the cutinase's enzymatic activity, wherein the pH indicator is selected from bromothymol blue, bromocresol purple, litmus, anthocyanin, and/or methyl red;
   - a thickening agent comprising sodium carboxymethyl cellulose (CMC) present in an amount of at least 0.05 to at most 5.0 wt.%; and,
   - a buffer and/or pH adjustment agent comprising sodium bicarbonate (NaHCO3) and/or sodium hydroxide (NaOH) present in a concentration of at least 1.0 mM to at most 5.0 mM;
   - an application means configured for applying the biodegradability composition on a polymeric material; and optionally,
   - a system, preferably a personal computing hardware such as a smartphone, a tablet, or a phablet, comprising an optical sensor unit configured for registering a degradation of the polymeric material by the composition, the system being configured for determining from data outputted by the sensor
unit a biodegradability type of the polymeric material.

In particular, the kit as disclosed herein is characterized in that the application means are chosen from an adhesive plaster and/or a handheld pumping system, pipette or syringe.

In particular, the kit as disclosed herein is characterized in that the application means is a 1 component (1K) system comprising application means configured for applying the biodegradability composition on a polymeric material wherein the application means is a handheld pumping system, spray bottles, spread-on bottle, roll-on bottle, pump-action marker pen, and the like.

In particular, the kit as disclosed herein is characterized in that the application means is a 2 component (2K) system comprising
- a first component for holding the cutinase in a dry powdered form or liquid concentrate and configured for placement on the polymeric material; and,
- a second component for holding the buffer and pH indicator in a liquid form and configured for depositing on the first component.

In particular, the kit as disclosed herein is characterized in that the application means is a 2 component (2K) system wherein the second component is a syringe dispensing tube, two-nib pens, pump-action marker pen, and the like.

The application of the composition comprised in the kit on the polymeric material may be performed in various ways, with or without tools. The application means might need to have certain properties such as good moisture and oxygen barrier properties. Moreover, the application means may need to be produced with a material that is not interactive with the cutinase during application. The skilled person is, however, well aware of such requirements and knows which materials may be combined to accomplish such requirements.

A particularly suitable example of a handheld pumping system includes the pump markers produced by Molotow; for example the 211EM empty marker model with a refillable body and a 4mm tip. The exemplary markers may allow for an improved easy-of-use, and the modularity may allow for easier refill or replacement of parts. Further suitable examples are handheld pumping systems which are multichannelled via a multitude of tips and corresponding reservoirs each containing a defined composition, adding up to a matrix of data that gives advanced information on the type of plastic and its degradation tendency and speed.

In some embodiments the kit comprises instructions for performing the method according to one or more embodiments as described herein. The instructions may be provided in various ways, for instance on a readable or illustrative medium. For example, an insert may be provided, or an illustrative guide. If the kit comprises a packaging, the packaging may comprise the instructions. Alternatively, reference may be made to an external medium, such as a link to a website. The instructions are not to be seen as limited by language or limited technical knowledge of the consumer.

In some embodiments the kit comprises a storage means or storage compartment; for example a packaging. The packaging may be provided in various materials, such as plastics or paper. The packaging might need to have certain properties such as good moisture and oxygen barrier properties. In order to arrive at the required properties a combination of different materials might be required, for example, in form of a two-layer or multi-layer film. The skilled person is, however, well aware of such requirements and knows which materials may be combined to accomplish such requirements.

In a further aspect, disclosed herein is a biodegradability composition for determining the biodegradability of polymeric materials comprising:
- 0.1 to 10.0 wt.% of a cutinase;
- a pH indicator suitable for visually indicating the cutinase's enzymatic activity, wherein the pH indicator is selected from bromothymol blue, bromocresol purple, litmus, anthocyanin, and/or methyl red;
- a thickening agent comprising sodium carboxymethyl cellulose (CMC) present in an amount of at least 0.05 to at most 5.0 wt.%; and,
- a buffer and/or pH adjustment agent comprising sodium bicarbonate (NaHCO3) and/or sodium hydroxide (NaOH) present in a concentration of at least 1.0 mM to at most 5.0 mM.

Preferably, the present invention relates to a hydrogel (biodegradability) composition comprising: a cutinase; a pH indicator suitable for visually indicating the cutinase's enzymatic activity, preferably bromothymol blue or bromocresol purple; a thickening agent, preferably sodium carboxymethyl cellulose (CMC); and, a buffer;

Foreseen or above described embodiments of the composition as part of or used in the method or the kit are also foreseen embodiments of the composition.

In a further aspect, disclosed herein is a method for producing the biodegradability composition, the method comprising the steps of:
(a) preparing an aqueous solution comprising at least one humectant; preferably glycerol and/or propylene glycol;
(b) adding a pH indicator to the solution of step (a); preferably bromothymol blue, litmus, bromocresol purple, anthocyanin, and/or methyl red;
(c) adding a preservative to the solution of step (b); preferably phenoxyethanol;
(d) adding a thickening agent to the solution of step (c); preferably poly(acrylic acid), hydroxyethyl cellulose (HEC), and/or sodium Carboxymethyl cellulose (CMC).
(e) adding a cutinase to the solution of step (d), optionally together with a stabilizer; preferably chosen from *Thermobifida cellulosilytica* cutinase (Thc-cutl), *Thermobifida alba* cutinase (F71X06) and/or *Fusarium solani* cutinase, in particular *Fusarium solani* pisi cutinase (Cut1-Fusso);
(f) adding a pH buffer solution to the solution of step (e); preferably NaOH and/or NaHCO₃.

In some embodiments step (e) may be performed prior to step (d). In some embodiments steps (d) and (e) may be performed simultaneously. In some embodiments the thickening agent in step (d) is added gradually, preferably sprinkled into the solution. In some embodiments the cutinase in step (e) is added gradually, preferably sprinkled into the solution.

In some embodiments, the cutinase is added in the form of a powder; preferably a freeze-dried or spray-dried enzyme powder. The powder may comprise processing additives, such as carbohydrate and/or salts.

The above production method enables a production of the biodegradability composition in a single sequence; the method is also referred to as the "all-in one" production method.

In a further aspect, disclosed herein is a method for producing the biodegradability composition, the method comprising the steps of:
(I) preparing a first component comprising water and a thickening agent;
   preferably the thickening agent is poly(acrylic acid), hydroxyethyl cellulose (HEC), and/or sodium Carboxymethyl cellulose (CMC).
(II) preparing a second component comprising a humectant, a pH indicator and a preservative;
   preferably the humectant is propylene glycol;
   preferably the pH indicator is bromothymol blue and/or bromocresol purple;
   preferably the preservative is phyenoxyethanol;
(III) preparing a third component comprising a pH buffer solution;
   preferably the pH buffer solution comprises NaOH and/or NaHCO₃;
(IV) mixing the (I) first, (II) second and (III) third component with a cutinase, optionally together with a stabilizer;
   preferably the stabilizer is glycerol;
   preferably the cutinase is chosen from *Thermobifida cellulosilytica* cutinase (Thc-cutl), *Thermobifida alba* cutinase (F71X06) and/or *Fusarium solani* cutinase, in particular *Fusarium solani* pisi cutinase (Cut1-Fusso).

In some embodiments the preparation steps of the (I) first, (II) second and (III) third component may be interchanged or performed simultaneously. In some embodiments the (I) first and the (II) second components are first mixed prior to addition of the (III) third component; preferably wherein the (I) first and the (II) second components are stirred and the (III) third component is titrated into the (I) first and (II) second component mixture. In a particular embodiment wherein the pH indicator is bromothymol blue, the (III) third component is titrated until a pH of about 8.0 to 8.2 is reached. In a particular embodiment wherein the pH indicator is bromocresol purple, the (III) third component is titrated until a pH of about 7.0 to 7.5 is reached.

In some embodiments, the cutinase is added in the form of a powder; preferably a freeze-dried (lyophilized) or spray-dried enzyme powder. The cutinase powder may comprise processing additives, such as carbohydrate and/or salts. Preferably the cutinase powder is added together with a stabilizer, such as glycerol, to improve the dispersion.

In particular, the method, kit and/or composition as disclosed herein is characterized in that the biodegradability composition further comprises glycerol or sorbitol typically in an amount ranging between 5 and 50 wt%, more preferably between 20 and 30 wt%, such as for instance 25 wt%.

In a further aspect, disclosed herein is the use of the composition or the kit as disclosed herein for determining the biodegradability of polymeric materials. Foreseen embodiments of the composition or the kit are also foreseen embodiments of the use of the composition or the kit.

In some embodiments (*Fusarium soloni*) cutinase is present in a concentration of at least 0.1 g/l to at most 50.0 g/l; preferably 1.0 g/l to 25.0 g/l; more preferably 2.0 g/l to 10.0 g/l; most preferably 3.0 g/l to 5.0 g/l; for example 4.0 g/l; for example 4.5 g/l.

In some embodiments the composition is used on a polymeric material is selected from the following list of dominantly aliphatic linear polyesters: polybutylene succinate (PBS), polybutylene adipate terephthalate (PBAT), polyhydroxyalkanoates (PHAS) such as poly-3-hydroxybutyrate (PHB), polyhydroxyvalerate (PHV) and polyhydroxyhexanoate (PHH), polylactic acid (PLA), polycaprolactone (PCL), polybutylene sebacate, polyanhydrides, polyvinyl alcohol, cellulose esters, such as cellulose acetate and nitrocellulose and their derivatives, and/or polyethylene terephthalate. In some embodiments the composition is used on polymeric materials that are starch filled.

In a further aspect, the present invention relates to a use of the kit according to one or more embodiments as described herein for determining the biodegradability of polymeric materials. Foreseen embodiments of the kit are also foreseen embodiments of the use of the kit.

### EXAMPLES

To better illustrate the properties, advantages and features of the present invention some preferred embodiments are disclosed as examples with reference to the enclosed figures. Accordingly, the present invention discloses many embodiments and adjustments as appreciated by those skilled in the art and the scope of the present invention is by no means limited to one the illustrative examples presented below.

### Example 1: testing the reliability of the biodegradability composition

Cutinase (*Fusarium solani* pisi) was overexpressed in *Pichia.* The supernatant was ultra-filtered and up-concentrated to 20 g/L. The cutinase solution was diluted 4 times and added to a 5mM NaHCO₃ buffered hydrogel containing bromothymol blue. After mixing, the hydrogel was applied onto a series of plastic substrates: namely: PP, LDPE, HDPE, PS, PC, PA-6, PBS and PBAT.

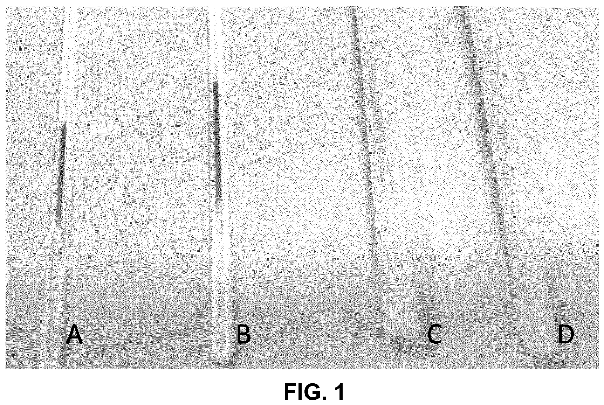

No color change was observed when the composition was applied to PP, LDPE, HDPE, PS, PC and PA-6; i.e. the composition remained blue (see Figure 1 A and B). In contrast, the blue color changed to light yellow (e.g. lowering pH) when applied to PBS (< 20 sec) and PBAT (< 80 sec) (see Figure 1 C and D).

In conclusion, the biodegradability composition was firstly able to distinguish the biodegradable plastics from the non-biodegradable ones; and secondly, it also was able to determine that PBS has a higher biodegradability rate compared to PBAT.

### Example 2: computer implemented diagnostic method

By way of an example, the computer implemented method as disclosed herein may perform the following operations:
1. CONFIRM by user (pushing NEW TEST button)
2. COMMAND by app: (CHECK) Is your plastic white, pure and dry?
3. CONFIRM by user (pushing YES button)
4. COMMAND by app: take pen, shake it in the air, and put three pumps on a tissue to give a fresh tip
5. CONFIRM by user (pushing DONE button)
6. COMMAND by app: Put a dot on your plastic with the tip - use the pump once to add extra liquid
7. CONFIRM by user (pushing DOTTED button) - at that moment the visible count-down of the app starts!
8. (direct response after pushing the DOTTED button:) COMMAND by app: "Now directly photograph the DOT in the center" (animated with SHOOT)
9. CRITERIUM by app: AGREE / non-agree that the plastic is white, pure and dry
10. CONFIRMATION is registered automatically by app upon taking the picture
11. From that point on, a timer starts to run with clear visible black numbers (00:01...)
12. (if there is more than 10 seconds between pushing the DOTTED button and the photo taken, the test is announced "abandoned", and user is invited to retake at stage 3)
13. After 15 seconds of timer (so at 00:15), there is an announcement: 5 seconds to go before next camera shot (counting down via 4, 3, 2, 1, SHOOT)
14. (if there is more than 10 seconds after the SHOOT moment to take the photo, a noise appears that supports the SHOOT command - the moment of registering can in fact always be fitted on the curve internally in the app) The overall timer just continues to run.
15. At 00:35, there is an announcement: 5 seconds to go before next camera shot (counting down via again via 4, 3, 2, 1, SHOOT)
16. (if there is more than 10 seconds after the SHOOT moment to take the photo, a noise appears that supports the SHOOT command - the moment of registering can in fact always be fitted on the curve internally in the app) The overall timer just continues to run.
17. At 00:55, there is an announcement: 5 seconds to go before next camera shot (counting down via again via 4, 3, 2, 1, SHOOT)
18. (if there is more than 10 seconds after the SHOOT moment to take the photo, a noise appears that supports the SHOOT command - the moment of registering can in fact always be fitted on the curve internally in the app) The overall timer just continues to run.
19. At 01:15, there is an announcement: 5 seconds to go before next camera shot (counting down via again via 4, 3, 2, 1, SHOOT)
20. (if there is more than 10 seconds after the SHOOT moment to take the photo, a noise appears that supports the SHOOT command - the moment of registering can in fact always be fitted on the curve internally in the app) The overall timer just continues to run.
21. At 01:35, there is an announcement: 5 seconds to go before next camera shot (counting down via again via 4, 3, 2, 1, SHOOT)
22. (if there is more than 10 seconds after the SHOOT moment to take the photo, a noise appears that supports the SHOOT command - the moment of registering can in fact always be fitted on the curve internally in the app) The overall timer just continues to run.
23. At 1:55, there is an announcement: 5 seconds to go before next camera shot (counting down via again via 4, 3, 2, 1, SHOOT)
24. (if there is more than 10 seconds after the SHOOT moment to take the photo, a noise appears that supports the SHOOT command - the moment of registering can in fact always be fitted on the curve internally in the app) The overall timer just continues to run.
25. READ-OUT by app:
   (After every camera shot, the app can decide to short-cut directly to stage 24 in case the yellow color has been reached convincingly and it makes no sense to wait for more yellowing)

Upon opening the app:
1. COMMAND by app: "Scan QR code of pen" (with camera)
2. Read-out the lifetime of the composition in the pen - a claim about the remaining time window to use the composition in the pen (read-out in days)

### Example 3: production and application of the biodegradability composition

By way of example, two methods of component incorporation are presented: (1) the 'all-in mixing' method, and (2) the 'mixing-in-parts' method. The 'all-in' mixing method involved mixing all components into the base water, in a sequential manner, with the liquid components being added first and the powders being added towards the end, and the buffer system added last to stabilise the composition. The 'mixing in parts' method involved creating a base hydrogel by first producing and subsequently mixing parts A, B, and C together, to which the enzyme powder, glycerol were added. The ordering of components is presented below in Table 1.

**Table 1: Ordering of components used to formulate the biodegradability composition.**

| **Component** | ***'All-in mixing'*** | ***'Mixing in parts'*** |
|---|---|---|
| Water | 1^{st} | Part A |
| pH indicator (e.g. bromothymol blue or bromocresol purple) | 4^{th} | Part B |
| Carboxymethyl cellulose | 6^{th} (sprinkled in gradually) | Part A (sprinkled in gradually) |
| Glycerol | 3^{rd} | Added to Part (A+B+C) mixture |
| Propylene glycol | 2^{nd} | Part B |
| Cutinase | 6^{th} (sprinkled in gradually) | Added to Part (A+B+C) mixture |
| Phenoxyethanol | 5^{th} | Part B |
| NaOH | 8^{th} | Part C |
| Optionally, NaHCO₃ | 7^{th} | Part C |

To achieve proper mixing using the 'all-in' mixing method the following mixing protocol was applied: Distilled water was first added to a large beaker - the li should only be half-filled to allow for space when the stirrer commences at high speed - and placed under an overhead stirrer fitted with a large propeller stirrer. Stirring was then started at ~500-600 rpm, and a vortex quickly formed. CMC (0.5 wt%) was then slowly, yet consistently, added to the mixture, with care taken to pour into the vortex, over the course of a two minute period; the speed was gradually increased in accordance with the added CMC and thus increasing viscosity up to a value of ~1,500 rpm. The solution was then left to stir for 10 minutes (or until no clumping was visible) at high speed to fully hydrate the CMC. After 10 minutes the stirrer was removed and the solution was allowed to stand for about 30 minutes to allow the dispersal of the tiny air bubbles that had formed. The product was a smooth, clear, semi-transparent solution, which forms Part A of the method.

Using Part A as a base matrix, Part B (consisting of a pH indicator, phenoxyethanol, and propylene glycol) was added and stirred using a magnetic stirrer. Finally, once fully mixed, NaOH and NaHCO₃ (Part C) were titrated in till the desired pH was reached. To this stable hydrogel, glycerol, enzyme powder and NaOH can be added as required. Depending on the pH indicator, NaHCO₃ may also be added. In particular, for bromocresol purple, NaHCO₃ can be left out while titrating the mixture a pH of about 7. However, for bromothymol blue, NaHCO₃ can be added while titrating to reach a pH of about 8. As a general rule, approximately 2 mmol of buffer (NaOH or NaHCO₃) was added per wt.% of cutinase. The cutinase was added in an amount of between 1--2 wt% (i.e. 10-20 g/L).

### Example 4: Biodegradability composition application method and kit

Using the 'mixing in parts' method described above in Example 3, two exemplary hydrogels were produced. The first hydrogel comprises bromothymol blue and is referred to as the 'blue' hydrogel; the second hydrogel comprises Bromocresol purple and is referred to as the 'purple' hydrogel. The cutinase can be added as required in powder form to each hydrogel. The composition of the two hydrogels is presented below in Table 2.

**Table 2: Composition of two exemplary biodegradability compositions.**

| **'Blue' hydrogel (pH 8)** | | **Wt%** | **'Purple' hydrogel (pH 7)** | | **Wt%** |
|---|---|---|---|---|---|
| 0.5% CMC solution | Part A | 94 | 0.5% CMC solution. | Part A | 94 |
| Bromothymol blue | Part B | 0.01 | Bromocresol purple | Part B | 0.04 |
| Propylene glycol | | 5 | Propylene glycol | | 5 |
| Phenoxyethanol | | 0.5 | Phenoxyethanol | | 0.5 |
| NaOH (500 mM soln.) | Part C | 0.1 | NaOH (100 mM soln.) | Part C | 1 |
| NaHCO₃ (100 mM soln) | | 0.5 | | | |

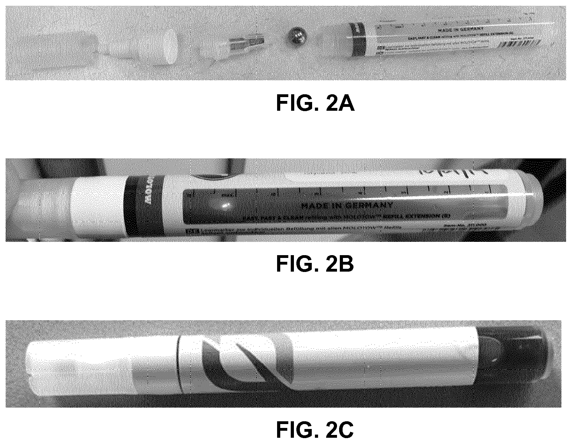

Using the produced 'Blue' and 'Purple' hydrogel formulations, exemplary application kits were assembled. Reference is made to FIG. 2, which illustrates a pump-action marker pen (from Molotow). In particular, FIG. 2A illustrates the modularity of the system, allowing easy filling of the biodegradability composition.

A suitable amount of cutinase (Cut1-Fusso) in dry powder (lyophilized) form produced using freeze drying was added to each of the two exemplary hydrogel composition, together with glycerol and optionally NaOH where required. In particular, a first marker pen was filled with the 'Blue' hydrogel to assemble a 'Blue' pen, which is illustrated in FIG.2B. A second first marker pen was filled with the 'Purple' hydrogel to assemble a 'Purple' pen, which is illustrated in FIG. 2C. The final compositions of the two marker pens is presented below in Table 3.

**Table 3: Final Composition of two exemplary biodegradability kits.**

| **'Blue' marker pen** | **Wt%** | **'Purple' marker pen** | **Wt%** |
|---|---|---|---|
| 'Blue' hydrogel (from Table 2) | 73.5% | 'Purple' hydrogel (from Table 2) | 73.5% |
| Glycerol | 25% | Glycerol | 25% |
| Cutinase (lyophilized powder) | 1.5% | Cutinase (lyophilized powder) | 1.5% |
| NaOH [500 mM] | 1.15% | NaOH [100 mM] | 2.75% |
| NaHCO₃ [100 mM] | 5% | | |

Using the 'Blue' and/or 'Purple' marker pens the biodegradability of a polymeric material is determined by marking the surface of said polymeric material with the marker, thereby releasing an amount of biodegradability composition onto the surface of said polymeric material. The biodegradability composition will interact with the polymeric material and visually indicate its biodegradability through a colour switch (depending on the pH indicator colour). The 'Blue' hydrogel has a working pH range of 8 to 6 pH, wherein the hydrogel transitions from blue to yellow responsive to the cutinase's enzymatic activity. The 'Purple' hydrogel has a working pH range of 7 to 5 pH, wherein the hydrogel transitions from purple to yellow responsive to the cutinase's enzymatic activity.

## Claims

1. A method for determining and/or tracing the biodegradability of a polymeric material, the method comprising the steps of:
(i) providing a hydrogel composition comprising:
- 0.05 to 10.0 wt.% of a cutinase;
- a pH indicator suitable for visually indicating the cutinase's enzymatic activity, wherein the pH indicator is selected from bromothymol blue,bromocresol purple, litmus, anthocyanin, and/or methyl red;
- a thickening agent comprising sodium carboxymethyl cellulose (CMC) present in an amount of at least 0.05 to at most 5.0 wt.%; and,
- a buffer and/or pH adjustment agent comprising sodium bicarbonate (NaHCO3) and/or sodium hydroxide (NaOH) present in a concentration of at least 1.0 mM to at most 5.0 mM;
(ii) applying the hydrogel composition on a polymeric material; and,
(iii) determining and/or tracing the biodegradability of said polymeric material responsive to the visual indication of the biodegradability composition.

2. The method according to claim 1, wherein the method is a computer-implemented method for determining the degradability of a polymeric material, and wherein step (iii) for determining and/or tracing the biodegradability comprises the steps of:
(a) receiving sensor data from an optical sensor unit configured for registering a degradation of the polymeric material by the hydrogel composition;
(b) computing, from the optical sensor unit data, a degradation rate of the polymeric material;
(c) computing, from the degradation rate, a degradability type of the polymeric material; and,
(d) optionally, displaying the degradability type of the polymeric material on a display unit.

3. A hydrogel composition for determining and/or tracing the degradability of polymeric materials, the composition comprising:
- 0.1 to 10.0 wt.% of a cutinase;
- a pH indicator suitable for visually indicating the cutinase's enzymatic activity, wherein the pH indicator is selected from bromothymol blue, bromocresol purple, litmus, anthocyanin, and/or methyl red;
- a thickening agent comprising sodium carboxymethyl cellulose (CMC) present in an amount of at least 0.05 to at most 5.0 wt.%; and,
- a buffer and/or pH adjustment agent comprising sodium bicarbonate (NaHCO3) and/or sodium hydroxide (NaOH) present in a concentration of at least 1.0 mM to at most 5.0 mM.

4. A kit for executing and/or facilitating the method according to any one of claims 1 or 2, the kit comprising:
- a hydrogel composition according to claim 3;
- an application means configured for applying the hydrogel composition on a polymeric material.

5. The kit according to claim 4, further comprising a computing system, preferably a personal computing hardware such as a smartphone, a tablet, or a phablet; the computing system comprising an optical sensor unit configured for registering a degradation of the polymeric material by the hydrogel composition, and optionally a display unit; the computing system being configured for determining from data outputted by the optical sensor unit a degradability type of the polymeric material.

6. The kit according to any one of claims 4 or 5, wherein the application means is a syringe dispensing tube, two-nib pen, or preferably a pump-action marker pen.

7. Use of the composition or the kit according to any one of claims 3 to 6, for determining the biodegradability of polymeric materials.

8. The method, composition, kit or use according to any one of claims 1 to 7, wherein the cutinase is *Thermobifida cellulosilytica* cutinase (Thc-cutl), *Thermobifida alba* cutinase (F71X06) and/or *Fusarium solani* cutinase (Cut1-Fusso); preferably *Fusarium solani* pisi cutinase.

9. The method, composition, kit or use according to any one of claims 1 to 8, wherein the cutinase is present in an amount of at least 0.05 to at most 5.0 wt.% of the total weight of the hydrogel composition; preferably 0.05 to 4.0 wt.%, preferably 0.05 to 3.0 wt.%, more preferably 0.1 to 2.0 wt.%; more preferably 0.1 to 1.0 wt.%, even more preferably 0.2 to 0.9 wt.%, even more preferably 0.3 to 0.8 wt.%, even more preferably 0.3 to 0.7 wt.%; for example 0.4 wt.%, for example 0.5 wt.%, for example 0.6 wt.%.

10. The method, composition, kit or use according to any one of claims 1 to 9, wherein sodium carboxymethyl cellulose (CMC) is present in an amount of at least 0.05 to at most 3.0 wt.%; preferably 0.1 to 2.0 wt.%; more preferably 0.1 to 1.0 wt.%; more preferably 0.2 to 0.7 wt.%, even more preferably 0.3 to 0.5 wt.%.

11. The method, composition, kit or use according to any one of claims 1 to 10, wherein the pH indicator is bromothymol blue, and the hydrogel composition has a pre-application pH of at least 8.0 to at most 8.5; preferably 8.1 to 8.4; more preferably 8.2 to 8.3; and/or,
wherein the pH indicator is bromocresol purple, and the hydrogel composition has a pre-application pH of 7.0 to 7.5; preferably 7.1 to 7.4; more preferably 7.2 to 7.3.

12. The method, composition, kit or use according to any one of claims 1 to 11, wherein the buffer nd/or pH adjustment agent comprises sodium bicarbonate (NaHCO₃) present in a concentration of 2.0 mM to 4.5 mM; more preferably 3.0 mM to 4.0 mM; most preferably about 3.0 mM; and/or, sodium hydroxide (NaOH) present in a concentration of 2.0 mM to 4.5 mM; more preferably 3.0 mM to 4.0 mM; most preferably about 4.0 mM.

13. The method, composition, kit or use according to any one of claims 1 to 12, wherein the hydrogel composition comprises a humectant, preferably propylene glycol; preferably present in at least 0.1 to at most 20.0 wt% of the total weight of the hydrogel composition; more preferably 1.0 to 10.0 wt%; more preferably 1.0 to 5.0 wt%; for example 3 wt%.

14. The method, composition, kit or use according to any one of claims 1 to 13, wherein the hydrogel composition comprises a stabilizer, preferably glycerol or sorbitol; preferably present in at least 5 to at most 50 wt% of the total weight of the hydrogel composition; more preferably 10 to 40 wt%; more preferably 20 to 30 wt%; for example 25 wt%.

15. The method, composition, kit or use according to any one of claims 1 to 14, wherein the hydrogel composition comprises a preservative, preferably phyenoxyethanol; preferably present in at least 0.05 to at most 2.0 wt% of the total weight of the hydrogel composition; preferably 0.1 to 1.5 wt%; preferably 0.1 to 1.0 wt%; more preferably 0.1 to 0.5 wt%.

## Patentansprüche

1. Verfahren zur Bestimmung und/oder Verfolgung der biologischen Abbaubarkeit eines Polymermaterials, wobei das Verfahren die Schritte umfasst:
(i) Bereitstellen einer Hydrogelzusammensetzung, umfassend:
- 0,05 bis 10,0 Gew.% einer Cutinase;
- einen pH-Indikator, der geeignet ist, um die enzymatische Aktivität der Cutinase visuell anzugeben, wobei der pH-Indikator ausgewählt ist aus Bromthymolblau, Bromcresolviolett, Lackmus, Anthocyan und/oder Methylrot;
- ein Verdickungsmittel, umfassend NatriumCarboxymethylcellulose (CMC), das in einer Menge von mindestens 0,05 bis höchstens 5,0 Gew.% vorhanden ist;
- einen Puffer und/oder ein pH-Werteinstellmittel, umfassend Natriumbicarbonat (NaHCO₃) und/oder Natriumhydroxid (NaOH), der bzw. das in einer Konzentration von mindestens 1,0 mM bis höchstens 5,0 mM vorhanden ist bzw. sind;
(ii) Applizieren der Hydrogelzusammensetzung auf ein Polymermaterial; und
(iii) Bestimmen und/oder Verfolgen der biologischen Abbaubarkeit des Polymermaterials in Reaktion auf die visuelle Angabe der biologischen Abbaubarkeitszusammensetzung.

2. Verfahren nach Anspruch 1, wobei das Verfahren ein rechnerimplementiertes Verfahren zum Bestimmen der Abbaubarkeit von Polymermaterial ist, und wobei Schritt (iii) zum Bestimmen und/oder Verfolgen der biologischen Abbaubarkeit die Schritte umfasst:
(a) Empfangen von Sensordaten von einer optischen Sensoreinheit, die zum Registrieren eines Abbaus des Polymermaterials durch die Hydrogelzusammensetzung ausgestaltet ist;
(b) Berechnen einer Abbaurate des Polymermaterials aus den Daten der optischen Sensoreinheit;
(c) Berechnen eines Abbaubarkeitstyps des Polymermaterials aus der Abbaurate; und
(d) gegebenenfalls Anzeigen des Abbaubarkeitstyps des Polymermaterials auf einer Anzeigeeinheit.

3. Hydrogelzusammensetzung zur Bestimmung und/oder Verfolgung der Abbaubarkeit von Polymermaterialien, wobei die Zusammensetzung umfasst:
- 0,1 bis 10,0 Gew.% einer Cutinase;
- einen pH-Indikator, der geeignet ist, um die enzymatische Aktivität der Cutinase visuell anzugeben, wobei der pH-Indikator ausgewählt ist aus Bromthymolblau, Bromcresolviolett, Lackmus, Anthocyan und/oder Methylrot;
- ein Verdickungsmittel, umfassend NatriumCarboxymethylcellulose (CMC), das in einer Menge von mindestens 0,05 bis höchstens 5,0 Gew.% vorhanden ist; und
- einen Puffer und/oder ein pH-Einstellmittel, umfassend Natriumbicarbonat (NaHCO₃) und/oder Natriumhydroxid (NaOH), der bzw. das in einer Konzentration von mindestens 1,0 mM bis höchstens 5,0 mM vorhanden ist bzw. sind.

4. Kit zum Ausführen und/oder Erleichtern des Verfahrens nach einem der Ansprüche 1 oder 2, wobei das Kit umfasst:
- eine Hydrogelzusammensetzung nach Anspruch 3;
- ein Applikationsmittel, das zum Applizieren der Hydrogelzusammensetzung auf ein Polymermaterial ausgestaltet ist.

5. Kit nach Anspruch 4, des Weiteren umfassend ein Rechensystem, vorzugsweise eine persönliche Rechenhardware, wie ein Smartphone, ein Tablet oder ein Phablet; wobei das Rechensystem eine optische Sensoreinheit, die zum Registrieren eines Abbaus des Polymermaterials durch die Hydrogelzusammensetzung ausgestaltet ist, und gegebenenfalls eine Anzeigeeinheit umfasst; wobei das Rechensystem konfiguriert ist, um aus Daten, die durch die optische Sensoreinheit ausgegeben werden, einen Abbaubarkeitstyp des Polymermaterials zu bestimmen.

6. Kit nach einem der Ansprüche 4 oder 5, wobei das Applikationsmittel ein Spritzenausgaberöhrchen, ein Doppelspitzenstift oder vorzugsweise ein Markierstift mit Pumpmechanismus ist.

7. Verwendung der Zusammensetzung oder des Kits nach einem der Ansprüche 3 bis 6 zum Bestimmen der biologischen Abbaubarkeit von Polymermaterialien.

8. Verfahren, Zusammensetzung, Kit oder Verwendung nach einem der Ansprüche 1 bis 7, wobei die Cutinase *Thermobifida cellulosilytica-Cutinase* (Thccut1), *Thermobifida alba*-Cutinase (F71X06) und/oder *Fusarium* solani-Cutinase (Cut1-Fusso) ist; vorzugsweise *Fusarium solani* pisi-Cutinase.

9. Verfahren, Zusammensetzung, Kit oder Verwendung nach einem der Ansprüche 1 bis 8, wobei die Cutinase in einer Menge von mindestens 0,05 bis höchstens 5,0 Gew.% des Gesamtgewichts der Hydrogelzusammensetzung vorhanden ist; vorzugsweise 0,05 bis 4,0 Gew.%, vorzugsweise 0,05 bis 3,0 Gew.%, bevorzugter 0,1 bis 2,0 Gew.%; bevorzugter 0,1 bis 1,0 Gew.%, noch bevorzugter 0,2 bis 0,9 Gew.%, noch bevorzugter 0,3 bis 0,8 Gew.%, noch bevorzugter 0,3 bis 0,7 Gew.%; beispielsweise 0,4 Gew.%, beispielsweise 0,5 Gew.%, beispielsweise 0,6 Gew.%.

10. Verfahren, Zusammensetzung, Kit oder Verwendung nach einem der Ansprüche 1 bis 9, wobei NatriumCarboxymethylcellulose (CMC) in einer Menge von mindestens 0,05 bis höchstens 3,0 Gew.%; vorzugsweise 0,1 bis 2,0 Gew.%; bevorzugter 0,1 bis 1,0 Gew.%; bevorzugter 0,2 bis 0,7 Gew.%, noch bevorzugter 0,3 bis 0,5 Gew.% vorhanden ist.

11. Verfahren, Zusammensetzung, Kit oder Verwendung nach einem der Ansprüche 1 bis 10, wobei der pH-Indikator Bromthymolblau ist und die Hydrogelzusammensetzung einen pH-Wert vor Applikation von mindestens 8,0 bis höchstens 8,5; vorzugsweise 8,1 bis 8,4; bevorzugter 8,2 bis 8,3 hat; und/oder wobei der pH-Indikator Bromcresolviolett ist und die Hydrogelzusammensetzung einen pH-Wert vor Applikation von 7,0 bis 7,5; vorzugsweise 7,1 bis 7,4; bevorzugter 7,2 bis 7,3 hat.

12. Verfahren, Zusammensetzung, Kit oder Verwendung nach einem der Ansprüche 1 bis 11, wobei der Puffer und/oder das pH-Werteinstellmittel Natriumbicarbonat (NaHCO₃), das in einer Konzentration von 2,0 mM bis 4,5 mM, bevorzugter 3,0 mM bis 4,0 mM, am meisten bevorzugt etwa 3,0 mM vorhanden ist, und/oder Natriumhydroxid (NaOH) umfasst, das in einer Konzentration von 2,0 mM bis 4,5 mM, bevorzugter 3,0 mM bis 4,0 mM, am meisten bevorzugt etwa 4,0 mM vorhanden ist.

13. Verfahren, Zusammensetzung, Kit oder Verwendung nach einem der Ansprüche 1 bis 12, wobei die Hydrogelzusammensetzung ein Feuchthaltemittel, vorzugsweise Propylenglykol umfasst, das vorzugsweise in mindestens 0,1 bis höchstens 20,0 Gew.% des Gesamtgewichts der Hydrogelzusammensetzung, bevorzugter 1,0 bis 10,0 Gew.%, bevorzugter 1,0 bis 5,0 Gew.%, beispielsweise 3 Gew.% vorhanden ist.

14. Verfahren, Zusammensetzung, Kit oder Verwendung nach einem der Ansprüche 1 bis 13, wobei die Hydrogelzusammensetzung einen Stabilisator, vorzugsweise Glycerin oder Sorbit umfasst, der vorzugsweise in mindestens 5 bis höchstens 50 Gew.% des Gesamtgewichts der Hydrogelzusammensetzung, bevorzugter 10 bis 40 Gew.%, bevorzugter 20 bis 30 Gew.%, beispielsweise 25 Gew.% vorhanden ist.

15. Verfahren, Zusammensetzung, Kit oder Verwendung nach einem der Ansprüche 1 bis 14, wobei die Hydrogelzusammensetzung ein Konservierungsmittel, vorzugsweise Phenoxyethanol umfasst, das vorzugsweise in mindestens 0,05 bis höchstens 2,0 Gew.% des Gesamtgewichts der Hydrogelzusammensetzung, vorzugsweise 0,1 bis 1,5 Gew.%, vorzugsweise 0,1 bis 1,0 Gew.%, bevorzugter 0,1 bis 0,5 Gew.% vorhanden ist.

## Revendications

1. Procédé pour la détermination et/ou le traçage de la biodégradabilité d'un matériau polymérique, le procédé comprenant les étapes de :
(i) mise à disposition d'une composition d'hydrogel comprenant :
- 0,05 à 10,0 % en poids d'une cutinase ;
- un indicateur de pH approprié pour indiquer visuellement l'activité enzymatique de la cutinase, l'indicateur de pH étant choisi parmi le bleu de bromothymol, le pourpre de bromocrésol, le tournesol, une anthocyanine, et/ou le rouge de méthyle ;
- un agent épaississant comprenant une carboxyméthylcellulose (CMC) sodique présente en une quantité d'au moins 0,05 à au plus 5,0% en poids ; et,
- un agent tampon et/ou d'ajustement du pH comprenant le bicarbonate de sodium (NaHCO₃) et/ou l'hydroxyde de sodium (NaOH) présent en une concentration d'au moins 1,0 mM à au plus 5,0 mM ;
(ii) application de la composition d'hydrogel sur un matériau polymérique ; et,
(iii) détermination et/ou traçage de la biodégradabilité dudit matériau polymérique en réponse à l'indication visuelle de la composition de biodégradabilité.

2. Procédé selon la revendication 1, le procédé étant un procédé implémenté par ordinateur pour la détermination de la dégradabilité d'un matériau polymérique, et l'étape (iii) pour la détermination et/ou le traçage de la biodégradabilité comprenant les étapes de :
(a) réception de données de capteur provenant d'une unité de capteur optique configurée pour enregistrer une dégradation du matériau polymérique par la composition d'hydrogel ;
(b) calcul, à partir des données de l'unité de capteur optique, une vitesse de dégradation du matériau polymérique ;
(c) calcul, à partir de la vitesse de dégradation, d'un type de dégradabilité du matériau polymérique ; et,
(d) éventuellement, affichage du type de dégradabilité du matériau polymérique sur une unité d'affichage.

3. Composition d'hydrogel pour la détermination et/ou le traçage de la dégradabilité de matériaux polymériques, la composition comprenant :
- 0,1 à 10,0 % en poids d'une cutinase ;
- un indicateur de pH approprié pour indiquer visuellement l'activité enzymatique de la cutinase, l'indicateur de pH étant choisi parmi le bleu de bromothymol, le pourpre de bromocrésol, le tournesol, une anthocyanine, et/ou le rouge de méthyle ;
- un agent épaississant comprenant une carboxyméthylcellulose (CMC) sodique présente en une quantité d'au moins 0,05 à au plus 5,0 % en poids ; et,
- un agent tampon et/ou d'ajustement du pH comprenant le bicarbonate de sodium (NaHCO₃) et/ou l'hydroxyde de sodium (NaOH) présent en une concentration d'au moins 1,0 mM à au plus 5,0 mM.

4. Kit pour exécuter et/ou faciliter le procédé selon l'une quelconque des revendications 1 et 2, le kit comprenant :
- une composition d'hydrogel selon la revendication 3 ;
- un moyen d'application configuré pour appliquer la composition d'hydrogel sur un matériau polymérique.

5. Kit selon la revendication 4, comprenant en outre un système informatique, préférablement un matériel informatique personnel tel qu'un smartphone, une tablette ou une phablette ; le système informatique comprenant une unité de capteur optique configurée pour enregistrer une dégradation du matériau polymérique par la composition d'hydrogel, et éventuellement une unité d'affichage ; le système informatique étant configuré pour déterminer, à partir des données émises par l'unité de capteur optique, un type de dégradabilité du matériau polymérique.

6. Kit selon l'une quelconque des revendications 4 et 5, le moyen d'application étant un tube de distribution par seringue, un stylo à deux plumes ou préférablement un stylo marqueur à pompe.

7. Utilisation de la composition ou du kit selon l'une quelconque des revendications 3 à 6 pour la détermination de la biodégradabilité de matériaux polymériques.

8. Procédé, composition, kit ou utilisation selon l'une quelconque des revendications 1 à 7, la cutinase étant une cutinase *Thermobifida cellulosilytica* (Thc-cutI), une cutinase *Thermobifida alba* (F71X06) et/ou une cutinase *Fusarium solani* (Cut1-Fusso) ; préférablement une cutinase *Fusarium solani* pisi.

9. Procédé, composition, kit ou utilisation selon l'une quelconque des revendications 1 à 8, la cutinase étant présente en une quantité d'au moins 0,05 à au plus 5,0 % en poids du poids total de la composition d'hydrogel ; préférablement de 0,05 à 4,0 % en poids, préférablement de 0,05 à 3,0 % en poids, plus préférablement de 0,1 à 2,0 % en poids ; plus préférablement de 0,1 à 1,0 % en poids, encore plus préférablement de 0,2 à 0,9 % en poids, encore plus préférablement de 0,3 à 0,8 % en poids, encore plus préférablement de 0,3 à 0,7 % en poids ; par exemple de 0,4 % en poids, par exemple de 0,5 % en poids, par exemple de 0,6 % en poids.

10. Procédé, composition, kit ou utilisation selon l'une quelconque des revendications 1 à 9, la carboxyméthylcellulose (CMC) sodique étant présente en une quantité d'au moins 0,05 à au plus 3,0 % en poids ; préférablement de 0,1 à 2,0 % en poids ; plus préférablement de 0,1 à 1,0 % en poids ; plus préférablement de 0,2 à 0,7 % en poids ; encore plus préférablement de 0,3 à 0,5 % en poids.

11. Procédé, composition, kit ou utilisation selon l'une quelconque des revendications 1 à 10, l'indicateur de pH étant le bleu de bromothymol, et la composition d'hydrogel possédant un pH de pré-application d'au moins 8,0 à au plus 8,5 ; préférablement de 8,1 à 8,4 ; plus préférablement de 8,2 à 8,3 ; et/ou,
l'indicateur de pH étant le pourpre de bromocrésol, et la composition d'hydrogel possédant un pH de pré-application de 7,0 à 7,5 ; préférablement de 7,1 à 7,4 ; plus préférablement de 7,2 à 7,3.

12. Procédé, composition, kit ou utilisation selon l'une quelconque des revendications 1 à 11, l'agent tampon et/ou d'ajustement du pH comprenant le bicarbonate de sodium (NaHCO₃) présent en une concentration de 2,0 mM à 4,5 mM ; plus préférablement de 3,0 mM à 4,0 mM ; le plus préférablement d'environ 3,0 mM ; et/ou, l'hydroxyde de sodium (NaOH) présent en une concentration de 2,0 mM à 4,5 mM ; plus préférablement de 3,0 mM à 4,0 mM ; le plus préférablement d'environ 4,0 mM.

13. Procédé, composition, kit ou utilisation selon l'une quelconque des revendications 1 à 12, la composition d'hydrogel comprenant un humectant, préférablement le propylèneglycol ; préférablement présent en une quantité d'au moins 0,1 à au plus 20,0 % en poids du poids total de la composition d'hydrogel ; plus préférablement de 1,0 à 10,0 % en poids ; plus préférablement de 1,0 à 5,0 % en poids ; par exemple de 3 % en poids.

14. Procédé, composition, kit ou utilisation selon l'une quelconque des revendications 1 à 13, la composition d'hydrogel comprenant un stabilisant, préférablement le glycérol ou le sorbitol ; préférablement présent en une quantité d'au moins 5 à au plus 50 % en poids du poids total de la composition d'hydrogel ; plus préférablement de 10 à 40 % en poids ; plus préférablement de 20 à 30 % en poids ; par exemple de 25 % en poids.

15. Procédé, composition, kit ou utilisation selon l'une quelconque des revendications 1 à 14, la composition d'hydrogel comprenant un conservateur, préférablement le phénoxyéthanol ; préférablement présent en une quantité d'au moins 0,05 à au plus 2,0 % en poids du poids total de la composition d'hydrogel ; préférablement de 0,1 à 1,5 % en poids ; préférablement de 0,1 à 1,0 % en poids ; plus préférablement de 0,1 à 0,5 % en poids.
